# EUROPÄISCHE PATENTANMELDUNG

(11) **EP 4 755 328 A1**
(43) Veröffentlichungstag der Anmeldung: **10.06.2026**
(21) Anmeldenummer: 24217261.7
(22) Anmeldetag: 03.12.2024
(51) Int. Cl.: A61B 18/12, A61B 18/00

(54) **HF-GENERATOR ZUR SPEISUNG VON EINEM ODER MEHREREN MEDIZINISCHEN INSTRUMENTEN**

(71) Anmelder: Erbe Elektromedizin GmbH, 72072 Tübingen (DE)
(72) Erfinder: Hatzfeld, Falk, 72072 Tuebingen (DE); Kegreiss, Marc, 72108 Rottenburg (DE); Sauter, Michael, 72461 Albstadt (DE)
(74) Vertreter: Rüger Abel Patentanwälte PartGmbB

(57) **Zusammenfassung**

Die Erfindung betrifft einen HF-Generator (10) zur Speisung von mindestens einem medizinischen Instrument. Der erfindungsgemäße HF-Generator (10) weist einen Ausgangsblock (11), einen Oszillatorblock (12), einen Kommunikationsblock (13) und einen Netzblock (14) auf. Der Ausgangsblock (11) ist dazu eingerichtet, das eine oder die mehreren medizinischen Instrumente mit einem HF-Strom zu versorgen. Der Oszillatorblock (12) ist dazu eingerichtet, den Ausgangsblock (11) über einen ersten Leistungskoppler (28) mit dem HF-Strom zu versorgen. Außerdem ist der Oszillatorblock (12) über einen ersten Datenkoppler (22) mit dem Ausgangsblock (11) verbunden. Der Kommunikationsblock (13) ist über einen zweiten Leistungskoppler (29) mit dem Ausgangsblock (11) und über einen zweiten Datenkoppler 35 mit dem Oszillatorblock (12) verbunden. Eine Besonderheit des erfindungsgemäßen HF-Generators besteht darin, dass der erste Leistungskoppler (28) und der erste Datenkoppler (22) eine höhere Isolationsspannung aufweisen als die übrigen Leistungskoppler (29, 30) und Datenkoppler (35), wodurch der Ausgangsblock 11 mit nur zwei Kopplern, die mit Maßnahmen zur Erhöhung ihrer Isolationsspannung versehen sind, gegenüber dem Oszillatorblock (12) bei gleicher Funktionalität und gleichbleibender Spannungsfestigkeit abgesichert ist.

## Beschreibung

Die Erfindung betrifft einen Hochfrequenzgenerator (HF-Generator) zur Speisung von mindestens einem medizinischen Instrument, insbesondere von mindestens einem HF-chirurgischen Instrument, das beispielsweise zum Schneiden, Koagulieren sowie gegebenenfalls zur Erzielung weiterer Gewebeeffekte von biologischem Gewebe eines menschlichen oder tierischen Patienten eingerichtet ist.

HF-Generatoren zur Speisung von einem oder mehreren medizinischen Instrumenten sind aus dem Stand der Technik allgemein bekannt.

Die WO 2004/030552 A1 beschreibt einen elektrochirurgischen Generator zum Speisen einem oder mehreren medizinischen Geräten. Die medizinischen Geräte sollen eine Einwirkung auf biologisches Gewebe eines Patienten ermöglichen, wie z.B. das Schneiden oder Koagulieren von Gewebe. Dazu werden die medizinischen Geräte zumindest vorübergehend am Patienten angebracht oder gelangen mit diesem in Kontakt. Daher ist es besonders wichtig sicherzustellen, dass kein unkontrollierter Strom von den medizinischen Geräten über den Patienten fließen kann. Der kapazitiv gegen Erde koppelnde Patient soll gegenüber dem Stromversorgungsnetz potentialfrei gehalten werden. Um dies zu gewährleisten, sieht die WO 2004/030552 A1 eine Vielzahl von Isolationsstellen jeweils zwischen dem Versorgungsnetz, der Steuereinheit und dem Oszillator des Generators vor, die mit einer erhöhten Spannungsfestigkeit ausgeführt sind. Die Maßnahmen zur Erzielung einer erhöhten Spannungsfestigkeit der einzelnen Isolationsstellen sind konstruktiv aufwendig. Eine Vielzahl solcher Isolationsstellen führt daher zu einem vergleichsweise hohen Aufwand für die Systemarchitektur. Darüber hinaus stellt bei mehreren parallel angeordneten Isolationsstellen, die beispielsweise fertigungsbedingt ungleiche Durchschlagfestigkeiten aufweisen, die Isolationsstelle mit der geringsten Durchschlagfestigkeit die Schwachstelle der Systemarchitektur dar, so dass die Systemsicherheit trotz vergleichsweise aufwendiger Systemarchitektur teilweise kritisch gesehen werden kann.

Davon ausgehend liegt der vorliegenden Erfindung die Aufgabe zugrunde, einen HF-Generator mit einem verbesserten und vereinfachten Isolationsschema bereitzustellen. Insbesondere sollen in dem HF-Generator weniger, oder wenigstens weniger besonders spannungsfeste, Isolationsstellen benötigt werden - dies bei zumindest gleichbleibender oder verbesserter Sicherheit.

Diese Aufgabe wird mit dem HF-Generator nach Anspruch 1 gelöst:

Der erfindungsgemäße HF-Generator dient zur Speisung von mindestens einem medizinischen Instrument, bei welchem es sich insbesondere um mindestens ein HF-chirurgisches Instrument handelt. Der erfindungsgemäße HF-Generator weist einen Ausgangsblock, einen Oszillatorblock, einen Kommunikationsblock und einen Netzblock auf.

Der Ausgangsblock ist dazu eingerichtet, das eine oder die mehreren medizinischen Instrumente mit einem HF-Strom zu versorgen. HF-Ströme sind dabei hochfrequente Wechselströme, die eine Frequenz oberhalb von 100 kHz, vorzugsweise oberhalb von 300 kHz aufweisen können, beispielsweise zwischen 300 kHz und 4 MHz. Der Ausgangsblock weist insbesondere eine Sensoreinheit auf, die dazu eingerichtet sind, Sensordaten von den HF-Strömen, wie z.B. Stromwerte, Spannungswerte, Scheinleistungswerte, Wirkleistungswerte und/oder Blindleistungswerte, aber auch Werte wie die komplexe Impedanz des Instruments oder des Gewebes, Änderung dieser und/oder Werte für die Linearität der komplexen Impedanz, zu bestimmen. Der Ausgangsblock kann außerdem eine Vorverarbeitungseinheit aufweisen, die dazu eingerichtet sein kann, die Sensordaten, vorzugsweise in Echtzeit, vorzuverarbeiten.

Der Oszillatorblock ist dazu eingerichtet, den Ausgangsblock über einen ersten Leistungskoppler mit dem HF-Strom zu versorgen. Außerdem ist der Oszillatorblock über einen ersten Datenkoppler mit dem Ausgangsblock verbunden. Der Oszillatorblock weist insbesondere eine HF-Steuereinheit auf, die dazu eingerichtet ist, die gewünschten Parameter des HF-Ströme und die Sensordaten des Ausgangsblocks zu empfangen und dieser HF-Ströme anhand der Parameter und der Sensordaten zu regeln.

Der Kommunikationsblock ist über einen zweiten Leistungskoppler mit dem Ausgangsblock und über einen zweiten Datenkoppler mit dem Oszillatorblock verbunden. Der Netzblock ist mit dem Oszillatorblock und über einen dritten Leistungskoppler mit dem Kommunikationsblock speisend verbunden.

Eine Besonderheit des erfindungsgemäßen HF-Generators besteht darin, dass der erste Leistungskoppler eine höhere Isolationsspannung als die übrigen Leistungskoppler, vorzugsweise alle übrigen Leistungskoppler, aufweist. Vorzugsweise weist auch der erste Datenkoppler eine höhere Isolationsspannung als die übrigen Datenkoppler, vorzugsweise alle übrigen Datenkoppler, auf. Die höheren Isolationsspannungen des ersten Leistungskopplers und des ersten Datenkopplers bedeuten, dass diese eine höheren Durchschlagfestigkeit gegenüber Spannungsspitzen und Potentialdifferenzen zwischen dem Netz, dem HF-Generator und dem Patienten besitzen.

Das erfindungsgemäße Isolationskonzept sieht eine klare Trennung zwischen dem Ausgangsblock und dem Oszillatorblock vor, um die Anzahl der Isolationsstellen, insbesondere die Anzahl der hoch spannungsfesten Isolationsstellen zu verringern. Der Oszillatorblock ist nur wenig bis gar nicht gegenüber dem Netzblock gesichert, während der Ausgangsblock gegenüber dem Oszillatorblock stark gesichert ist - d.h. die Isolationsstellen zwischen Ausgangsblock und Oszillatorblock weisen eine hohe Isolationsspannung bzw. Isolationsfestigkeit auf.

Hierdurch kann der Ausgangsblock mit nur zwei hoch spannungsfesten Kopplern - einem (ersten) Leistungskoppler und einem (ersten) Datenkoppler - gegenüber dem Oszillatorblock, bei einem erhöhten Grad an Durchschlagsfestigkeit, isoliert werden. Das Isolationsschema des HF-Generators kann so wesentlich vereinfacht werden. Eingang und Ausgang des ersten Leistungskopplers und des ersten Datenkopplers sind voneinander jeweils galvanisch mit hoher Durchschlagfestigkeit getrennt. Die Durchschlagfestigkeit eines Kopplers ist dabei durch die maximale Isolationsspannung bzw. Durchschlagspannung definiert, die zwischen seiner Primärseite und seiner Sekundärseite anliegen kann, ohne dass es zu einem Spannungsdurchschlag bzw. Stromfluss kommt. Der erste Leistungskoppler kann beispielsweise ein Transformator mit getrennten, voneinander isolierten Wicklungen sein, die voneinander galvanisch und räumlich getrennt sind. Der erste Leistungskoppler weist eine besonders hohe elektrische Isolationsfestigkeit zwischen Primär- und Sekundärseite auf. Z.B. kann dies durch Unterbringung der Primär- und Sekundärwicklungen in getrennten, gekapselten Isolierstoffkammern des ersten Leistungskopplers erreicht werden. Durch die Reduktion auf lediglich einen Leistungskoppler mit erhöhter Sicherheit gegen Spannungsdurchschlag (erhöhte Isolationsfestigkeit) kann der technische Aufwand für den HF-Generator bei gleichbleibender oder gar gesteigerter Sicherheit verringert werden.

Vorzugsweise ist die Isolationsspannung, auf die der erste Leistungskoppler und der erste Datenkoppler ausgelegt sind, größer als die Summe der doppelten ausgangsseitigen Spitzenspannung des Oszillatorblocks und einer zusätzlichen Spannung, die als maximale Spannungsspitze auf der Betriebsspannung des Oszillatorblocks liegen kann. Diese Spannungsspitze ist die Summe aus der Betriebsgleichspannung des Oszillatorblocks und der doppelten Netzspitzenspannung, wobei diese Summe noch mit einem Sicherheitsfaktor multipliziert ist. Der Sicherheitsfaktor kann beispielsweise mindestens ein 2, 3, 4 oder mehr betragen. Der relativ groß bemessene Sicherheitsfaktor vermindert die Gefahr, dass der Wechselstrom des Netzblocks über den Oszillatorblock auf den Ausgangsblock und damit über eines der angeschlossenen medizinischen Instrumente auf den Patienten und/oder den Operateur überschlagen kann.

Für alle Koppler gilt grundsätzlich: Ein Leistungskoppler ist dazu eingerichtet, eine Leistung zwischen zwei Blöcken zu übertragen, wobei die beiden Blöcke galvanisch voneinander getrennt sind. Ein Datenkoppler ist dazu eingerichtet, Daten - also Informationen - zwischen zwei Blöcken zu übertragen, wobei beide Blöcke ebenfalls galvanisch voneinander getrennt sind.

Zwischen dem Ausgangsblock und dem Oszillatorblock ist insbesondere ein erster Isolationspfad gebildet, in welchem der erste Leistungskoppler und der erste Datenkoppler parallel zueinander angeordnet sind. Ein Isolationspfad ist ein denkbarer, jedoch durch eine oder mehrere isolierende Barrieren unterbrochener Weg, auf dem kein Strom von einem Ende zu dem anderen Ende des Isolationspfads fließen kann und darf. Dazu weisen die eine oder mehreren im Isolationspfad befindlichen Barrieren jeweils eine definierte Isolationsspannung auf, die die Isolationsspannung des Isolationspfades bestimmt. Der erste Leistungskoppler und der erste Datenkoppler weisen vorzugsweise eine gleich große Isolationsspannung auf.

Zwischen dem Ausgangsblock und dem Oszillationsblock ist vorzugsweise außerdem ein zweiter Isolationspfad gebildet, in welchem der zweite Isolationskoppler und der zweite Datenkoppler in Reihe angeordnet sind, wodurch sich die Isolationsspannungen des zweiten Leistungskopplers und des zweiten Datenkopplers entlang des zweiten Isolationspfads aufaddieren.

Der erste Isolationspfad und der zweite Isolationspfad sind insbesondere zueinander parallel angeordnet. Die Isolationsspannungen des ersten Isolationspfads und des zweiten Isolationspfads sind vorzugsweise gleich groß.

Insbesondere ist die Isolationsspannung des ersten Leistungskopplers und des ersten Datenkopplers jeweils größer, vorzugsweise wesentlich größer, als die Isolationsspannung des zweiten Leistungskopplers und des zweiten Datenkopplers - z.B. eineinhalb, zweimal oder zweieinhalbmal so groß.

Es wird bevorzugt, dass die aufsummierte Isolationsspannung des zweiten Leistungskopplers und des zweiten Datenkopplers der Isolationsspannung des ersten Leistungskopplers entspricht, vorzugsweise mit dieser übereinstimmt. Ebenso wird bevorzugt, dass die aufsummierte Isolationsspannung des zweiten Leistungskopplers und des zweiten Datenkopplers der Isolationsspannung des ersten Datenkopplers entspricht, vorzugsweise mit dieser übereinstimmt. Dies führt dazu, dass der erste Isolationspfad und der zweite Isolationspfad gleiche Isolationsspannungen aufweisen, wobei durch die parallele Anordnung des ersten Isolationspfads zum zweiten Isolationspfad bei einer Überspannung keiner der Isolationspfade nachgibt, sondern beide die Spannungsspitze ohne Spannungsdurchschlag überstehen.

Der erste und der zweite Datenkoppler sind vorzugsweise als induktive oder kapazitive Datenkoppler oder als Optokoppler ausgebildet. Der erste und der zweite sowie der dritte Leistungskoppler können hingegen jeweils als Transformator ausgebildet sein.

Vorzugsweise weist der Oszillatorblock eine HF-Einheit, eine HF-Steuereinheit, eine Netzeinheit und eine zweite Dateneinheit auf. Die HF-Steuereinheit ist insbesondere dazu eingerichtet, die HF-Einheit derart anzusteuern, dass die HF-Einheit HF-Ströme erzeugt, die über den ersten Leistungskoppler an den Ausgangsblock übertragen werden. Die HF-Ströme können unterschiedliche variierbare HF-Eigenschaften aufweisen, die im Betrieb des HF-Generators durch die HF-Steuereinheit geändert werden können. Die HF-Eigenschaften können dabei beispielsweise unterschiedliche Stromwerte, Spannungswerte, Frequenzwerte, Wellenformen, Crest-Faktoren, Taktungen, und dergleichen umfassen, um verschiedene Operationsmoden festzulegen.

Die Netzeinheit ist insbesondere dazu eingerichtet, die HF-Einheit von dem Netzblock kommend mit Netzleistung zu speisen. Die Netzeinheit kann eine Leistungsfaktor-Korrektureinheit aufweisen, um den netzseitig gezogenen Strom der Sinusform anzunähern und seine Oberschwingungen zu reduzieren. Die HF-Einheit kann dann dazu eingerichtet sein, die Leistungsfaktor-Korrektureinheit der Netzeinheit gegebenenfalls auch voreilend zu steuern, um immer, gerade auch bei Lastsprüngen, immer ausreichend Leistung bereitstellen zu können.

Der Ausgangsblock weist vorzugsweise eine Verteilereinheit auf. Diese ist dazu eingerichtet, die über den ersten Leistungskoppler erhaltene und z.B. hochtransformierte HF-Spannung auf das eine oder die mehreren medizinischen Instrumente zu verteilen. Die HF-Spannung kann z.B. mehr als 2 kV, 3 kV oder 4 kV betragen.

Die Verteilereinheit weist insbesondere mindestens eine Sensoreinheit auf, die zur Erfassung der HF-Ströme als Sensordaten im Ausgangsblock eingerichtet ist. Die Sensordaten können z.B. mindestens Stromwerte, Spannungswerte, Scheinleistungswerte, Wirkleistungswerte und/oder Blindleistungswerte umfassen.

Der Ausgangsblock kann außerdem eine Vorverarbeitungseinheit aufweisen, die mit der Sensoreinheit kommunikativ verbunden ist. Die Vorverarbeitungseinheit kann im einfachsten Fall z.B. lediglich einen Analog-/Digitalwandler umfassen, mit dem die analogen Sensordaten zu digitalen Sensordaten umgewandelt werden können. Die Vorverarbeitungseinheit kann aber auch dazu eingerichtet sein, komplexere Vorverarbeitungsschritte durchführen, wie z.B. die Bestimmung der komplexen Gewebeimpedanz, der Änderung der komplexen Gewebeimpedanz und/oder die Bestimmung eines Linearitätswertes der komplexen Gewebeimpedanz. Die Vorverarbeitungseinheit ist vorzugsweise dazu eingerichtet, die Sensordaten in Echtzeit zu bearbeiten.

Der Ausgangsblock kann zudem eine erste Dateneinheit aufweisen, die zur Zwischenspeicherung der (digitalen) Sensordaten und zur Bereitstellung Sensordaten für den Oszillatorblock eingerichtet ist. Die erste Dateneinheit ist insbesondere über den ersten Datenkoppler mit der zweiten Dateneinheit kommunikativ verbunden, so dass die Sensordaten zwischen der ersten Dateneinheit und der zweiten Dateneinheit ausgetauscht werden können.

Der Kommunikationsblock kann eine Bediensteuereinheit und eine mit der Bediensteuereinheit verbundene Kommunikationsschnittstelle zu mehreren Bedien- und Anzeigeeinheiten aufweisen. Über die Bedien- und Anzeigeeinheiten kann der Benutzer des HF-Generators, also z.B. ein Operateur, ein Chirurg, ein Assistenzarzt, oder eine OP-Hilfe, gewünschte Parameter für die HF-Steuereinheit vorgeben.

Die Bediensteuereinheit ist über den zweiten Datenkoppler mit der zweiten Dateneinheit kommunikativ verbunden, so dass die zweite Dateneinheit die vorgegebenen Parameter für die HF-Steuereinheit über die zweite Datenschnittstelle von der Bediensteuereinheit empfangen kann. Darüber hinaus kann die zweite Dateneinheit über den ersten Datenkoppler, die erfassten Sensordaten des Ausgangsblocks empfangen und zwischenspeichern. Die vorgegebenen Parameter und die erfassten Sensordaten können an die HF-Steuereinheit weitergegeben werden, die dazu eingerichtet ist, die HF-Einheit derart anzusteuern, dass die HF-Ströme im Ausgangsblock mit den vorgegebenen HF-Eigenschaften (Parametern) vorliegen und auf diese geregelt sind.

Vorzugsweise sind der erste Leistungskoppler und der erste Datenkoppler einer ersten Isolationsklasse zugeordnet, in der die Koppler (besonders) hohen Spannungen standhalten, wie z.B. 8 kV, 10 kV, 12 kV oder mehr. Die übrigen Leistungs- und Datenkoppler sind hingegen einer zweiten Isolationsklasse zugeordnet, in der die Koppler im Verhältnis lediglich niedrigeren Spannungen standhalten, wie z.B. 4 kV, 5 kV, 6 kV oder weniger - ohne, dass es zu einem Spannungsdurchschlag zwischen der Primärseite und der Sekundärseite kommt.

Der Ausgangsblock kann über den zweiten Leistungskoppler und den dritten Leistungskoppler gegenüber dem Netzblock gesichert sein.

Gemäß der erfindungsgemäßen Systemarchitektur des HF-Generators sind die Blöcke des HF-Generators gegenüber dem Netzblock in verschiedene Isolationszonen unterteilt, beispielsweise in eine Hochisolationszone, eine Zwischenisolationszone und eine Niederisolationszone. Die Hochisolationszone ist am besten gegen Spannungsdurchschlag infolge einer Spannungsspitze geschützt, die sich aus der Addition einer netzseitigen Spannungsspitze und einer vom HF-Generator erzeugten Spannung ergibt. Der Ausgangsblock befindet sich in der Hochisolationszone, der Kommunikationsblock in der Zwischenisolationszone und der Oszillatorblock in der Niederisolationszone.

Weitere Einzelheiten vorteilhafter Ausführungsformen der Erfindung ergeben sich aus den Unteransprüchen, aus den Zeichnungen oder aus der Beschreibung. Es zeigen:
Figur 1 ein Beispiel des erfindungsgemäßen HF-Generators, in schematischer Darstellung;
Figur 2 ein Isolationsschaubild des Isolationskonzepts für den erfindungsgemäßen HF-Generator;
Figur 3 ein weiteres Isolationsschaubild des Isolationskonzepts für den erfindungsgemäßen HF-Generator;
Figur 4 ein Beispiel des ersten Datenkopplers und des ersten Leistungskopplers;
Figur 5 ein weiteres Beispiel des ersten Datenkopplers und des ersten Leistungskopplers;
Figur 6 ein Beispiel der Netzeinheit im Oszillatorblock; sowie
Figur 7 ein Beispiel der HF-Einheit zusammen mit dem Oszillatorblock und der Anbindung an den Ausgangsblock.

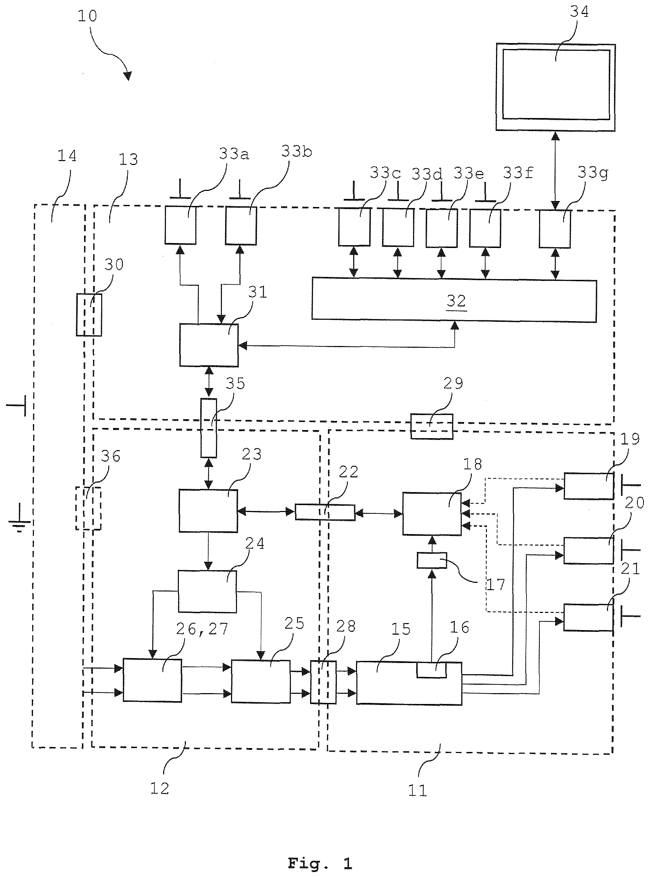

Figur 1 zeigt eine beispielhafte Darstellung des erfindungsgemäßen HF-Generators 10. Der HF-Generator 10 weist einen Ausgangsblock 11, einen Oszillatorblock 12, einen Kommunikationsblock 13 und einen Netzblock 14 auf.

Der Ausgangsblock 11 dient zur Versorgung der medizinischen Instrumente mit HF-Strömen, die im Oszillatorblock 12 erzeugt werden. Der Oszillatorblock 12 wird vom Netzblock 14 mit Netzspannung versorgt. Der Kommunikationsblock 13 dient als Schnittstelle zum Bediener des HF-Generators 10, über die der Bediener die im Oszillatorblock 12 erzeugten HF-Ströme einstellen kann.

Der Ausgangsblock 11 weist eine Verteilereinheit 15 mit einer Sensoreinheit 16, einer Vorverarbeitungseinheit 17 sowie einer ersten Dateneinheit 18 auf. Außerdem weist der in Figur 1 dargestellte Ausgangsblock 11 eine erste Instrumentenschnittstelle 19, eine zweite Instrumentenschnittstelle 20 und eine Neutralelektrodenschnittstelle 21 auf.

Die erste Instrumentenschnittstelle 19 und die zweite Instrumentenschnittstelle 20 sind zum Anschließen eines ersten und zweiten medizinischen Instruments an den Ausgangsblock 11 des HF-Generators 10 eingerichtet. Die Neutralelektrodenschnittstelle 21 ist zum Anschließen einer Neutralelektrode eingerichtet, die an einem Patienten angebracht werden kann. Die medizinischen Instrumente können monopolare und/oder bipolare HF-chirurgische Instrumente sein. Die Instrumenten- und Neutralelektrodenschnittstellen 19, 20, 21 werden von der Verteilereinheit 15 mit HF-Strömen versorgt. Die Instrumenten- und Neutralelektrodenschnittstellen 19, 20, 21 können dazu eingerichtet sein, festzustellen, ob ein medizinisches Instrument angeschlossen ist, und dies an die erste Dateneinheit zu kommunizieren. Die Instrumenten- und Neutralelektrodenschnittstellen 19, 20, 21 können außerdem dazu eingerichtet sein, angeschlossene Instrumente zu identifizieren.

Die Sensoreinheit 16 ist dazu eingerichtet, Sensordaten von den erzeugten HF-Strömen zu erfassen. Beispielsweise können diese Sensordaten Stromwerte, Spannungswerte, Leistungswerte, komplexe Impedanzen und dergleichen umfassen. Die Sensoreinheit 16 ist mit der Vorverarbeitungseinheit 17 verbunden.

Die Vorverarbeitungseinheit 17 kann in der einfachsten Form ein Analog-/Digitalwandler sein, der die von der Sensoreinheit 16 empfangenen, analogen Sensordaten, diskretisiert. Die Vorverarbeitungseinheit 17 kann aber auch dazu eingerichtet sein, komplexere Vorverarbeitungsschritte durchzuführen, mit denen die Sensordaten, möglichst in Echtzeit, vorverarbeitet werden können. Z.B. können die Sensorwertverläufe mit einem gleitenden Mittelwertfilter geglättet werden. Andere Vorverarbeitungen der Sensordaten, wie z.B. eine Rauschunterdrückung, Normalisierung der Daten, Filterung und dergleichen sind ebenfalls möglich. Die Vorverarbeitungseinheit 17 ist mit der ersten Dateneinheit 18 verbunden und zur Weiterleitung der digitalen Sensordaten an die erste Dateneinheit 18 eingerichtet.

Alternativ kann auch die Sensoreinheit 16 bereits einen Analog-/Digitalwandler umfassen. In diesem Fall kann die Sensoreinheit 16 direkt mit der ersten Dateneinheit 18 verbunden sein. Die digitalen Sensordaten können direkt von der Sensoreinheit 16 an die erste Dateneinheit 18 weitergeleitet werden.

Die erste Dateneinheit 18 ist zur Zwischenspeicherung der digitalen Sensordaten eingerichtet. Die erste Dateneinheit 18 ist über einen ersten Datenkoppler 22 mit dem Oszillatorblock 12 verbunden. Genauer gesagt ist der die erste Dateneinheit 18 über den ersten Datenkoppler 22 mit einer zweiten Dateneinheit 23 des Oszillatorblocks 12 kommunizierend verbunden.

Der Oszillatorblock 12 weist die zweite Dateneinheit 23, eine HF-Steuereinheit 24, eine HF-Einheit 25 und eine Netzeinheit 26 auf.

Die Netzeinheit 26 wird vom Netzblock 14 mit Netzspannung versorgt. Bei der Netzspannung handelt es sich um die je nach Land, in welchem der HF-Generator 10 betrieben wird, übliche Netzwechselspannung - z.B. um eine sinusförmige Wechselspannung mit einem Effektivwert von 230 V zwischen Außenleiter und Neutralleiter und einer Netzfrequenz von 50 Hz. Die Netzeinheit 26 kann eine Leistungsfaktorkorrektureinheit 27 aufweisen, mit welcher der Leistungsfaktor des Oszillatorblocks 12 erhöht und damit die störenden Oberschwingungen für das Netz reduziert werden können. Die Leistungsfaktorkorrektureinheit 27 wird von der HF-Steuereinheit 24 angesteuert.

Die Netzeinheit 26 kann dazu eingerichtet sein, die Netzspannung gleichzurichten und eine erzeugte Gleichspannung an die HF-Einheit 25 weiterzugeben. Die erzeugte Gleichspannung kann höher oder auch niedriger sein als die Netzspannung des Netzblocks. Die Netzeinheit 26 kann zum Umsetzen der gleichgerichteten Netzspannung z.B. einen Auswärtswandler aufweisen - der auch als Hochsetzsteller bezeichnet wird.

In der HF-Einheit 25 ist eine Oszillatorschaltung untergebracht, mit der aus der gleichgerichteten, hochgesetzten Gleichspannung ein hochfrequentes Spannungssignal erzeugt wird. Aus dem HF-Spannungssignal werden, z.B. mittels einer vorzugsweise im Schaltbetrieb arbeitenden Endstufe, HF-Ströme erzeugt, mit denen die medizinischen Instrumente versorgt werden. Die HF-Einheit 25 wird dabei von der HF-Steuereinheit 24 gesteuert.

Die erzeugten HF-Ströme der HF-Einheit 25 werden über den ersten Leistungskoppler 28 von dem Oszillatorblock 12 auf den Ausgangsblock 11 übertragen. Der erste Leistungskoppler 28 kann dabei als Teil der Endstufe zur Erzeugung der HF-Ströme dienen.

Das erfindungsgemäße Isolationskonzept des HF-Generators 10 sieht vor, dass der erste Leistungskoppler 28 und der erste Datenkoppler 22 im Vergleich zu den übrigen Daten- und Leistungskopplern des HF-Generators 10 eine wesentlich höhere Isolationsspannung aufweist, durch die die Durchschlagfestigkeit eines Bauteils definiert ist. Dies führt dazu, dass der Ausgangsblock 11 effektiv davor geschützt werden kann, dass Spannungsspitzen vom Netzblock 14 über den Oszillatorblock 12 auf den Ausgangsblock 11 durchschlagen. Die Isolationsspannung des ersten Datenkopplers und des ersten Leistungskopplers kann z.B. doppelt so groß sein, wie die Isolationsspannung der übrigen Daten- und Leistungskoppler sein. Durch die erhöhte Isolation des Ausgangsblocks 11 von dem Oszillatorblock 12 ist es möglich, die Anzahl von Isolationsstellen zwischen den Blöcken zu verringern und damit die Gesamtarchitektur des HF-Generators 10 zu vereinfachen.

Der Kommunikationsblock 13 weist einen zweiten Leistungskoppler 29 und einen dritten Leistungskoppler 30 auf. Der zweite Leistungskoppler 29 und der dritte Leistungskoppler 30 weisen eine geringere Isolationsspannung als der erste Leistungskoppler 28 auf. Beispielsweise entspricht die Summe der Isolationsspannungen des zweiten Leistungskoppler 29 und des dritten Leistungskopplers 30 der Isolationsspannung des ersten Leistungskopplers 28. Der zweite und der dritte Leistungskoppler 29, 30 dienen dazu, die von den verbundenen Blöcken, also dem Kommunikationsblock 13 und dem Ausgangsblock 11 umfassten Einheiten mit einer Betriebsspannung zu versorgen.

Der Kommunikationsblock 13 weist eine Bediensteuereinheit 31 auf, die mit einer Kommunikationsschnittstelle 32 verbunden ist, die eine Vielzahl von Bedien- und Anzeigeschnittstellen 33a bis 33g umfasst. Die Bedien- und Anzeigeschnittstellen 33a bis 33g können beispielsweise eine Lautsprecherschnittstelle 33a, eine Pedalschnittstelle 33b, weitere Hilfseingangsschnittstellen 33c, 33d, 33e, 33f (z.B. *Universeller Serieller Bus* (USB) oder andere Kommunikationsbusse) sowie eine Bildschirmschnittstelle 33g umfassen. Die Bedien- und Anzeigeschnittstellen 33a bis 33g ermöglichen es dem Bediener z.B. über eine Bedieneinheit, wie einem *Touchscreen,* Betriebsparameter für den HF-Generator 10 einzugeben sowie (aktuelle) Sensordaten, Betriebsparameter oder dergleichen auszugeben. Dies kann z.B. über eine Anzeigeeinheit 34 erfolgen, die einen Bildschirm aufweist. Die Steuerung des Kommunikationsblocks 13 erfolgt über die Bediensteuereinheit 31.

Die Bediensteuereinheit 31 ist über einen zweiten Datenkoppler 35 mit der zweiten Dateneinheit 23 verbunden. Auch der zweite Datenkoppler 35 weist eine geringere Isolationsspannung als der erste Datenkoppler 22 auf. Der erste Datenkoppler 22 und der zweite Datenkoppler 35 können beispielsweise als Optokoppler ausgebildet sein.

Zwischen dem Oszillatorblock 12 und dem Netzblock 14 kann außerdem mindestens ein vierter Leistungskoppler 36 angeordnet sein. Der vierte Leistungskoppler 36 kann eine wesentlich geringere Isolationsspannung als die anderen Leistungskoppler 28, 29, 30 aufweisen. Im Betrieb besteht keine Gefahr, dass der Patient, Bediener, der Operateur mit dem Oszillatorblock 12 in Kontakt kommt, wodurch wesentlich geringere Anforderungen an die Durchschlagsfestigkeit zwischen Netzblock 14 und Oszillatorblock 12 gestellt werden können. Wenn ein vierter Leistungskoppler 36 vorgesehen ist, kann dieser dazu dienen, die von dem Oszillatorblock 12 umfassten Einheiten mit einer Betriebsspannung zu versorgen. Alternativ können die Einheiten des Oszillatorblocks 12 auch direkt von dem Netzblock 14 versorgt werden.

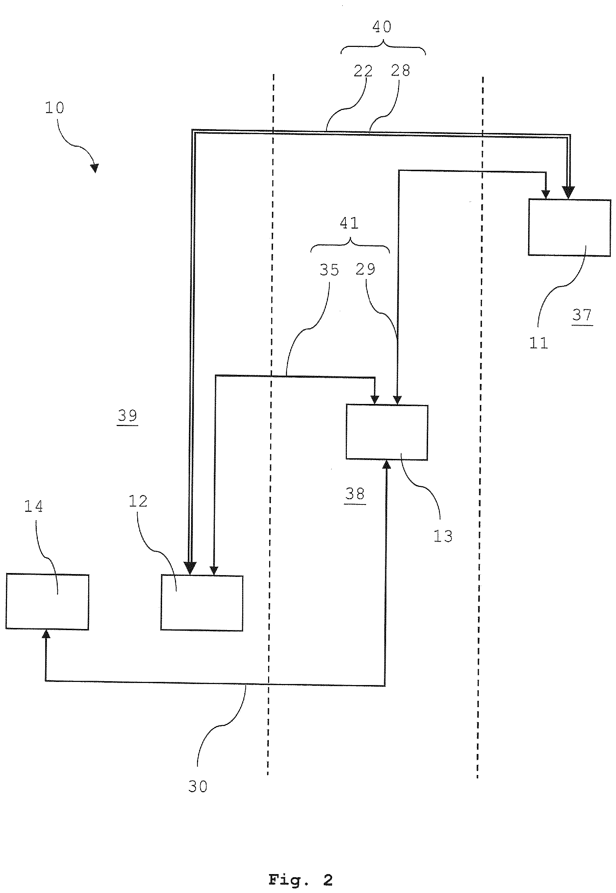

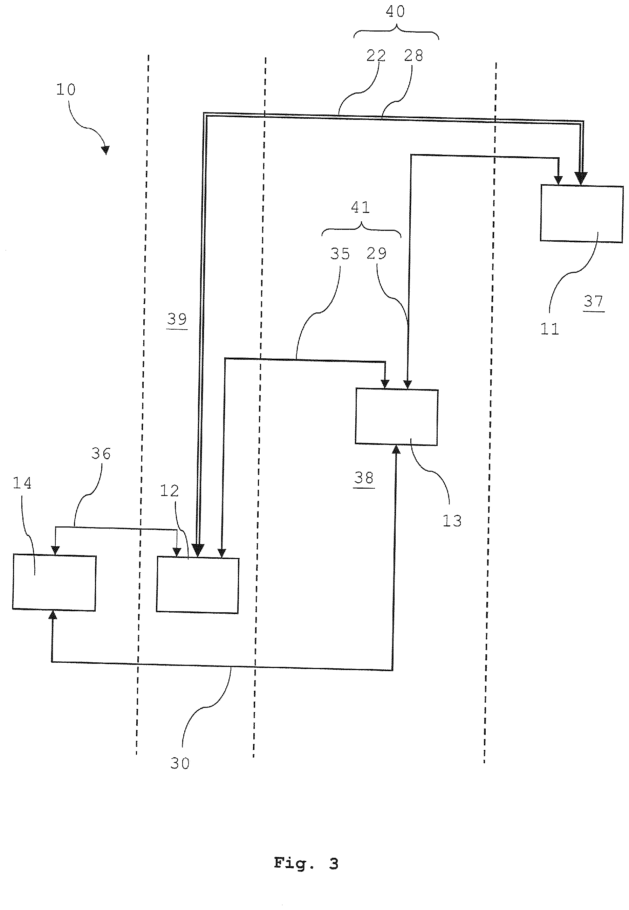

Zwischen den einzelnen Blöcken 11, 12, 13 und 14 des HF-Generators 10 sind über den ersten Leistungskoppler 28, dem zweiten Leistungskoppler 29, dem dritten Leistungskoppler 30, dem vierten Leistungskoppler 36, im ersten Datenkoppler 22 und im zweiten Datenkoppler 35, Isolationspfade definiert, die nachfolgend anhand von Figur 2 und Figur 3 näher erläutert werden.

Figur 2 und Figur 3 veranschaulichen das Isolationsschema des erfindungsgemäßen HF-Generators 10. In den Figuren 2 und 3 stellt der vertikale Abstand zwischen den Blöcken - also in Richtung der Höhe - die Isolationsspannungen der einzelnen Leistungs- und Datenkoppler zwischen einander dar.

In beiden in Figur 2 und in Figur 3 gezeigten Beispielen sind die Isolationsspannungen des ersten Leistungskopplers 28 und des ersten Datenkopplers 22 jeweils doppelt so groß wie die des zweiten Datenkopplers 35 und des zweiten Leistungskopplers 29.

Der HF-Generator 10 ist in mehrerer Isolationszonen, nämlich in einer Hochisolationszone 37, eine Zwischenisolationszone 38 und eine Niederisolationszone 39 aufgeteilt.

In dem in Figur 2 gezeigten Beispiel sind der Oszillatorblock 12 und der Netzblock 14 beide der Niederisolationszone 39 zugeordnet. Das heißt, dass zwischen beiden Blöcken keine oder nur eine vernachlässigbar kleine Isolationsspannung besteht. Der Ausgangsblock 11 ist der Hochisolationszone 37 zugeordnet. Der Kommunikationsblock 13 ist hingegen der Zwischenisolationszone 38 zugeordnet.

Zwischen dem Ausgangsblock 11 und dem Oszillatorblock 12 ist ein erster Isolationspfad 40 ausgebildet, in welchem der erste Datenkoppler 22 und der erste Leistungskoppler 28 parallel zueinander angeordnet sind.

Zwischen dem Ausgangsblock 11 und dem Oszillatorblock 12 besteht außerdem ein zweiter Isolationspfad 41, der über den zweiten Leistungskoppler 29, den Kommunikationsblock 13 und den zweiten Datenkoppler 35 (in Reihe) führt. Der erste Isolationspfad 40 und der zweite Isolationspfad 41 sind wiederrum zueinander parallel angeordnet. Darüber hinaus ist der Kommunikationsblock 13 gegenüber dem Netzblock 14 mittels des dritten Leistungskopplers 30 schutzisoliert.

Figur 3 veranschaulicht ein weiteres Isolationsschema für das erfindungsgemäße HF-Generator 10. Für Figur 3 gilt hinsichtlich der bereits eingeführten Bezugszeichen das zuvor Beschriebene entsprechend. Das Beispiel der Figur 3 unterscheidet sich von dem Beispiel der Figur 2 dadurch, dass zwischen dem Netzblock 14 und dem Oszillatorblock 12 ein vierter Leistungskoppler 36 angeordnet ist.

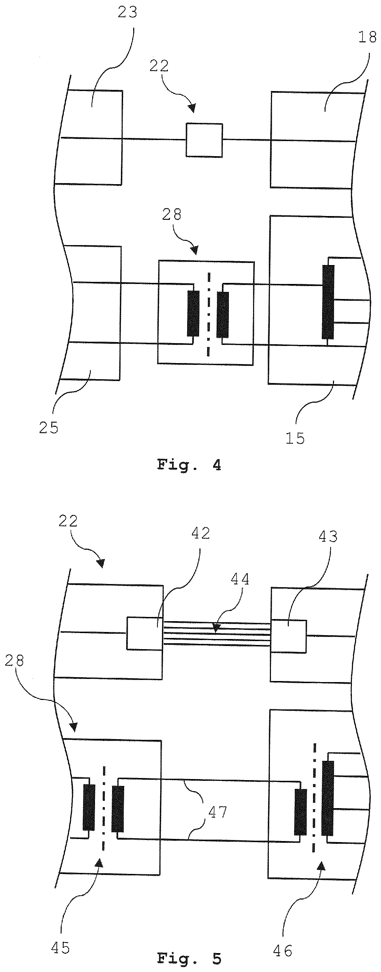

Figur 4 zeigt eine Detailansicht eines Beispiels für den ersten Datenkoppler 22 und den ersten Leistungskoppler 28. Der erste Datenkoppler 22 ist in diesem Beispiel als ein Optokoppler ausgebildet. Der erste Leistungskoppler 28 ist in dem gezeigten Beispiel als ein Transformator ausgebildet. Die Isolationsspannung des Transformators beschreibt seine Beständigkeit gegen einen Spannungsdurchschlag zwischen seiner Primärspule und seiner Sekundärspule. Der Transformator ist z.B. mit einer Isolationsspannung von 12 kV ausgelegt. Das bedeutet, dass eine Differenzspannung zwischen der Primärseite und der Sekundärseite des Transformators bis zu 12 kV betragen kann, ohne dass es zu einem Kurzschluss zwischen der Primärseite und der Sekundärseite des Transformators kommt. Primär- und Sekundärseite sind somit sicher elektrisch voneinander getrennt. Sekundärseitig ist in dem in Figur 4 gezeigten Beispiel ein Spartransformator zur Realisierung von einer oder mehrerer Abgriffe dargestellt, aus denen die medizinischen Instrumente versorgt werden können.

Figur 5 zeigt eine Detailansicht eines alternativen Beispiels für den ersten Datenkoppler 22 und den ersten Leistungskoppler 28. In diesem Beispiel umfasst der erste Datenkoppler 22 einen oszillatorblockseitigen Optokoppler 42 und einen ausgangsblockseitigen Optokoppler 43, die über mehrere optische Verbindungsleitungen 44 miteinander verbunden sind.

In dem in Figur 4 gezeigten Beispiel ergibt sich die Isolationsspannung des ersten Datenkopplers 22 aus der Summe der Isolationsspannung des oszillatorblockseitigen Optokopplers 42 und des ausgangsblockseitigen Optokopplers 43. Beispielsweise kann der oszillatorblockseitige Optokoppler 42 eine Isolationsspannung von 6 kV und der ausgangsblockseitige Optokoppler 43 ebenfalls eine Isolationsspannung von 6 kV aufweisen. Insgesamt weist dann der erste Datenkoppler 22 eine Isolationsspannung von 12 kV auf.

Der erste Leistungskoppler 28 weist einen oszillatorblockseitigen Transformator 45 und einen ausgangsblockseitigen Transformator 46 auf. Die Sekundärseite des oszillatorblockseitigen Transformators 45 ist mit der Primärseite des ausgangsblockseitigen Übertragungstransformators 46 über Übertragungsleitung 47 verbunden.

Der oszillatorblockseitige Übertragungstransformator 45 und der ausgangsblockseitige Übertragungstransformator 46 sind in diesem Beispiel jeweils für eine Isolationsspannung ausgelegt, welche sich durch die Reihenschaltung der beiden Transformatoren aufaddieren. Beispielsweise beträgt die Isolationsspannung der beiden 6 kV, so dass der erste Leistungskoppler 28 insgesamt eine Isolationsspannung von 12 kV aufweist. Bei dieser Ausführungsform ist es vorteilhaft, wenn die beiden Übertragungstransformatoren 45, 46 jeweils miteinander übereinstimmende parasitäre Kapazitäten zwischen der jeweiligen Primärspule und der jeweiligen Sekundärspule aufweisen. Dies obwohl die beiden Transformatoren 45, 46 aufgrund der geforderten Spannungsüber- oder Untersetzung naturgemäß nicht baugleich sein können. Infolge der übereinstimmenden parasitären Kapazitäten teilt sich aber eine Spannungsspitze zwischen der Primärseite des Transformators 45 und der Sekundärseite des Transformators 46 zu gleich großen Teilen auf die beiden Transformatoren 45, 46 auf. Damit wird ein serieller Spannungsdurchschlag durch den Leistungskoppler 28 vermieden. Gegebenenfalls können zu den parasitären Kapazitäten ein oder mehrere Kondensatoren parallel geschaltet werden, um das genannte Spannungsgleichgewicht herbeizuführen.

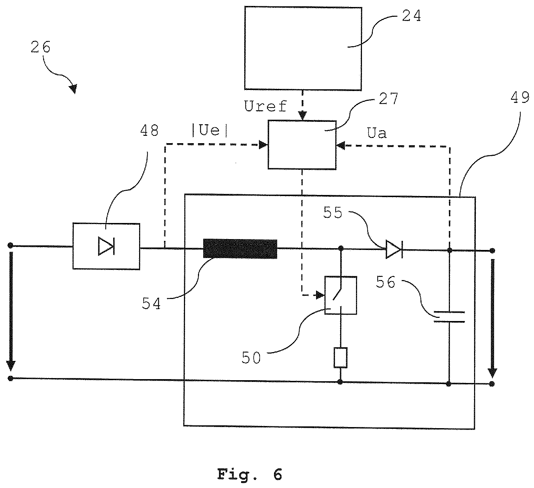

Figur 6 veranschaulicht die Netzeinheit 26 des Oszillatorblocks 12. Bei dem in Figur 6 gezeigten Beispiel umfasst die Netzeinheit 26 einen Gleichrichter 48, der eine eingangsseitige Netzspannung in eine Gleichspannung wandelt. Die Netzeinheit 26 weist außerdem einen Aufwärtswandler 49 auf, mit dem die erzeugte Gleichspannung hochgesetzt werden kann.

Der Aufwärtswandler 49 weist einen Schalter 50, eine Spule 54, eine Diode 55 und eine Kondensator 56 auf. Aufgrund der Induktivität der Spule 54 bleibt der Stromfluss aufrechterhalten, wenn der Schalter 50 geöffnet ist. Die Spannung an dem ausgangsseitigen Ende steigt daher rasch an, bis sie die am Kondensator 56 anliegende Spannung überschritten ist und somit die Diode 55 leitet. Der Strom fließt so im ersten Moment unverändert weiter und lädt den Kondensator 56 weiter auf. Das Magnetfeld der Spule wird dabei abgebaut und gibt seine Energie ab, indem es den Strom über die Diode 55 in den Kondensator 56 treibt. Die Kapazität des Kondensators 56 ist so ausgelegt, dass die Ausgangsspannung während eines Arbeitszyklus näherungsweise konstant ist.

Der Schalter 50 wird von der Leistungsfaktor-Korrektureinheit 27 angesteuert. Die Leistungsfaktor-Korrektureinheit 27 erhält den Betrag der gleichgerichteten Netzspannung (der Eingangsspannung Ue) und die Ausgangsspannung (Ua) des Aufwärtswandlers 49 sowie eine Referenzspannung Uref von der HF-Steuereinheit 24. In der Leistungsfaktor-Korrektureinheit 27 wird die Differenz aus Ausgangsspannung Ua und der Referenzspannung mit dem Betrag der Eingangsspannung Ue multipliziert, um einen Sollstrom zu berechnen, mit dem der Schalter 50 angesteuert wird. Der Leistungsfaktor kann so auf einen Wert nahe bei 1 eingestellt werden.

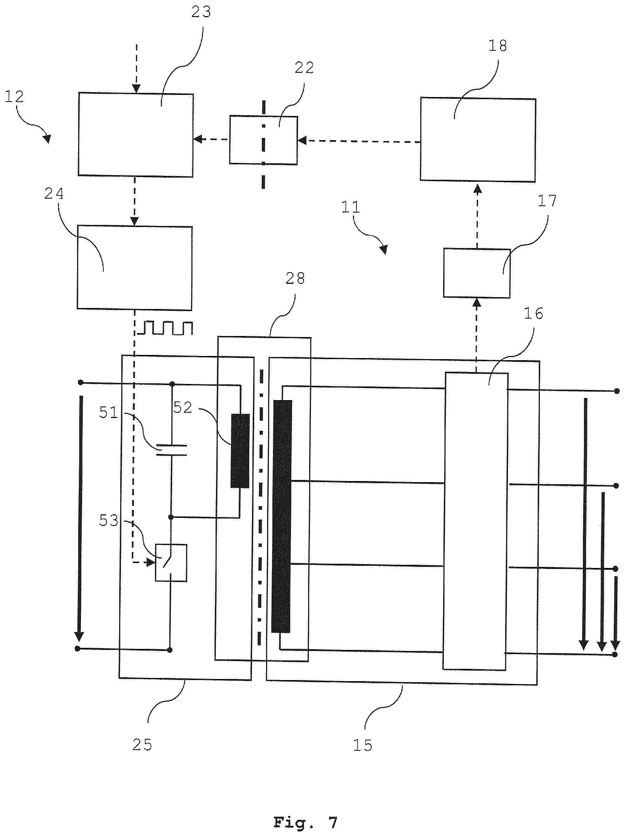

Figur 7 zeigt eine Detailansicht der HF-Einheit 25 mit dem ersten Leistungskoppler 28 und der Verteilereinheit 15 des Ausgangsblocks 11. Die HF-Einheit 25 weist eine Oszillatorschaltung auf, die einen Kondensator 51, eine Spule 52 und einen Schalter 53 aufweist. Der Schalter 53 wird von der HF-Steuereinheit 24 angesteuert.

Die Spule 52 der HF-Einheit 25 kann bereits eine Seite des ersten Leistungskopplers 28 sein, welche beispielsweise als Transformator ausgebildet ist. In Figur 7 ist die Spule 52 die Primärwicklung des Transformators des ersten Leistungskopplers 28. Auf der Sekundärseite des ersten Leistungskopplers 28 schließt sich die Verteilereinheit 15 an. Die Sekundärwicklung des Transformators kann hierbei ebenfalls dazu verwendet werden, verschiedene HF-Ströme mit unterschiedlichen Spannungspegeln abzugreifen. In der Verteilereinheit 15 ist eine Sensoreinheit 16 untergebracht, mit der die ausgangsseitig anliegenden Ströme, Spannungen, komplexe Widerstände und dergleichen, erfassbar sind. Die Sensoreinheit 16 ist mit der Vorverarbeitungseinheit 17 verbunden, die die Sensordaten vorverarbeitet und über die erste Dateneinheit mittels des ersten Datenkopplers an die Steuereinheit 24 weitergibt.

Die Erfindung betrifft einen HF-Generator 10 zur Speisung von mindestens einem medizinischen Instrument, insbesondere von mindestens einem HF-chirurgischen Instrument, das beispielsweise zum Schneiden, Koagulieren sowie gegebenenfalls zur Erzielung weiterer Gewebeeffekte an biologischem Gewebe eines menschlichen oder tierischen Patienten eingerichtet ist. Der erfindungsgemäße HF-Generator 10 weist einen Ausgangsblock 11, einen Oszillatorblock 12, einen Kommunikationsblock 13 und einen Netzblock 14 auf. Der Ausgangsblock 11 ist dazu eingerichtet, das eine oder die mehreren medizinischen Instrumente mit einem HF-Strom zu versorgen. Der Oszillatorblock 12 ist dazu eingerichtet, den Ausgangsblock 11 über einen ersten Leistungskoppler 28 mit dem HF-Strom zu versorgen. Außerdem ist der Oszillatorblock 12 über einen ersten Datenkoppler 22 mit dem Ausgangsblock 11 verbunden. Der Kommunikationsblock 13 ist über einen zweiten Leistungskoppler 29 mit dem Ausgangsblock 11 und über einen zweiten Datenkoppler 35 mit dem Oszillatorblock 12 verbunden. Eine Besonderheit des erfindungsgemäßen HF-Generators besteht darin, dass der erste Leistungskoppler 28 und der erste Datenkoppler 22 eine höhere Isolationsspannung aufweisen als die übrigen Leistungskoppler 29, 30 und Datenkoppler 35, wodurch der Ausgangsblock 11 mit nur zwei Kopplern, die mit Maßnahmen zur Erhöhung ihrer Isolationsspannung versehen sind, gegenüber dem Oszillatorblock 12 bei gleicher Funktionalität und gleichbleibender Spannungsfestigkeit abgesichert ist.

### Bezugszeichenliste

- 10: HF-Generator
- 11: Ausgangsblock
- 12: Oszillatorblock
- 13: Kommunikationsblock
- 14: Netzblock
- 15: Verteilereinheit
- 16: Sensoreinheit
- 17: Vorverarbeitungseinheit
- 18: erste Dateneinheit
- 19: erste Instrumentenschnittstelle
- 20: zweite Instrumentenschnittstelle
- 21: Neutralelektronenschnittstelle
- 22: erster Datenkoppler
- 23: zweite Dateneinheit
- 24: HF-Steuereinheit
- 25: HF-Einheit
- 26: Netzeinheit
- 27: Leistungsfaktorkorrektureinheit
- 28: erster Leistungskoppler
- 29: zweiter Leistungskoppler
- 30: dritter Leistungskoppler
- 31: Bediensteuereinheit
- 32: Kommunikationsschnittstelle
- 33a-33g: Bedien- und Ausgabeschnittstellen
- 34: Anzeigeeinheit (Bildschirm)
- 35: zweiter Datenkoppler
- 36: vierter Leistungskoppler
- 37: Hochisolationszone
- 38: Zwischenisolationszone
- 39: Niederisolationszone
- 40: erster Isolationspfad
- 41: zweiter Isolationspfad
- 42: Oszillatorblockseitiger Optokoppler
- 43: Ausgangsblockseitiger Optokoppler
- 44: Optische Verbindungsleitung
- 45: Oszillatorblockseitiger Übertragungstransformator
- 46: Ausgangsblockseitiger Übertragungstransformator
- 47: Übertragungsleitung
- 48: Gleichrichter
- 49: Aufwärtswandler
- 50: Schalter des Aufwärtswandlers
- 51: Kondensator der Oszillatorschaltung
- 52: Spule der Oszillatorschaltung
- 53: Schalter der Oszillatorschaltung
- 54: Spule des Aufwärtswandlers
- 55: Diode des Aufwärtswandlers
- 56: Kondensator des Aufwärtswandlers
- Ue: Eingangsspannung des Aufwärtswandlers
- Ua: Ausgangsspannung des Aufwärtswandlers
- Uref: Referenzspannung

## Patentansprüche

1. HF-Generator (10) zur Speisung von einem oder mehreren medizinischen Instrumenten, insbesondere von einem oder mehreren HF-chirurgischen Instrumenten, aufweisend:
- einen Ausgangsblock (11), der dazu eingerichtet ist, das eine oder die mehreren medizinischen Instrumente mit einem HF-Strom zu versorgen,
- einen Oszillatorblock (12), der dazu eingerichtet ist, den Ausgangsblock (11) über einen ersten Leistungskoppler (28) mit dem HF-Strom zu versorgen, wobei der über einen ersten Datenkoppler (22) mit dem Ausgangsblock (11) verbunden ist;
- einen Kommunikationsblock (13), der über einen zweiten Leistungskoppler (29) mit dem Ausgangsblock (11) und über einen zweiten Datenkoppler (35) mit dem Oszillatorblock (12) verbunden; und
- einen Netzblock (14), der mit dem Oszillatorblock (12) und über einen dritten Leistungskoppler (30) mit dem Kommunikationsblock (13) speisend verbunden ist, wobei der erste Leistungskoppler (28) eine höhere Isolationsspannung als die übrigen Leistungskoppler (29, 30) aufweist.

2. HF-Generator (10) nach Anspruch 1, **dadurch gekennzeichnet, dass** der erste Datenkoppler (22) eine höhere Isolationsspannung als der zweite Datenkoppler (35) aufweist.

3. HF-Generator (10) nach Anspruch 1 oder 2, **dadurch gekennzeichnet, dass** zwischen dem Ausgangsblock (11) und dem Oszillatorblock (12) ein erster Isolationspfad (40) gebildet ist, in welchem der erste Leistungskoppler (28) und der erste Datenkoppler (22) parallel zueinander angeordnet ist.

4. HF-Generator (10) nach einem der vorhergehenden Ansprüche, **dadurch gekennzeichnet, dass** zwischen dem Ausgangsblock (11) und dem Oszillatorblock (12) ein zweiter Isolationspfad (41) gebildet ist, in welchem der zweite Leistungskoppler (29) und der zweite Datenkoppler (35) in Reihe angeordnet sind.

5. HF-Generator (10) nach einem der vorhergehenden Ansprüche, **dadurch gekennzeichnet, dass** die Isolationsfestigkeit des ersten Leistungskopplers (28) und des ersten Datenkopplers (22) jeweils zumindest gleich oder größer als die Isolationsfestigkeit des zweiten Leistungskopplers (29) und des zweiten Datenkopplers zusammen ist.

6. HF-Generator (10) nach einem der vorhergehenden Ansprüche, **dadurch gekennzeichnet, dass** der erste und der zweite Datenkoppler (22, 35) als induktive oder kapazitive Datenkoppler oder als Optokoppler (42, 43) ausgebildet sind.

7. HF-Generator (10) nach einem der vorhergehenden Ansprüche, **dadurch gekennzeichnet, dass** der Oszillatorblock (12) eine HF-Einheit (25), eine HF-Steuereinheit (24), eine Netzeinheit, und eine zweite Dateneinheit (23) aufweist,
wobei die HF-Steuereinheit (24) dazu eingerichtet ist, die HF-Einheit (25) derart anzusteuern, dass diese HF-Ströme mit unterschiedlichen Parametern erzeugt, wobei die Parameter beispielsweise unterschiedliche Stromwerte, Spannungswerte, Wellenformen, Crest-Faktoren, Taktungen, Moden und dergleichen.

8. HF-Generator (10) nach einem der vorhergehenden Ansprüche, **dadurch gekennzeichnet, dass** die Netzeinheit (26) ein Leistungsfaktorkorrektureinheit (27) aufweist, wobei die HF-Steuereinheit (24) dazu eingerichtet ist, den Leistungsfaktor mittels des Leistungsfaktorkorrektureinheit (27) anzupassen.

9. HF-Generator (10) nach einem der vorhergehenden Ansprüche, **dadurch gekennzeichnet, dass** der Ausgangsblock (11) eine Verteilereinheit (15) aufweist, die dazu eingerichtet ist, den über den ersten Leistungskoppler (28) erhaltenen HF-Strom auf das eine oder die mehreren Instrumente zu verteilen.

10. HF-Generator (10) nach einem der vorhergehenden Ansprüche, **dadurch gekennzeichnet, dass** die Verteilereinheit (15) mindestens eine Sensoreinheit (16) aufweist, die zur Erfassung der HF-Ströme als Sensordaten im Ausgangsblock (11) eingerichtet ist, wobei die Sensordaten vorzugsweise mindestens Strom-, Spannungs-, Scheinleistungs-, Wirkleistungs-, und/oder Blindleistungsmesswerte, besonders bevorzugt eine komplexe Impedanz des Gewebes, Änderungen der komplexen Impedanz des Gewebes, und/oder einen Linearitätswert der komplexen Impedanz des Gewebes umfassen.

11. HF-Generator (10) nach einem der vorhergehenden Ansprüche, **dadurch gekennzeichnet, dass** der Ausgangsblock (11) eine erste Dateneinheit (18) aufweist, die mit der Sensoreinheit kommunikativ verbunden und zur Verteilung und Zwischenspeicherung der Sensordaten eingerichtet ist.

12. HF-Generator (10) nach einem der vorhergehenden Ansprüche, **dadurch gekennzeichnet, dass** die erste Dateneinheit (18) mit der zweiten Dateneinheit (23) über den ersten Datenkoppler (22) kommunikativ verbunden ist.

13. HF-Generator (10) nach einem der vorhergehenden Ansprüche, **dadurch gekennzeichnet, dass** der Kommunikationsblock (13) eine Bediensteuereinheit (31) und eine oder mehrere mit der Bediensteuereinheit (31) verbundene Bedien- und Anzeigeschnittstellen (33a,...,33g) aufweist, über die der Anwender Parameter für die HF-Steuereinheit (24) eingeben kann.

14. HF-Generator (10) nach einem der vorhergehenden Ansprüche, **dadurch gekennzeichnet, dass** die Bediensteuereinheit (31) über den zweiten Datenkoppler (35) mit der zweiten Dateneinheit (23) kommunikativ verbunden ist.

15. HF-Generator (10) nach einem der vorhergehenden Ansprüche, **dadurch gekennzeichnet, dass** die Blöcke (11, 12, 13) gegenüber dem Netzblock (14) verschiedenen Isolationszonen (37, 38, 39) mit unterschiedlichen Isolationsspannungen zugeordnet sind, beispielsweise in eine Hochisolationszone (37), eine Zwischenisolationszone (38) und eine Niederisolationszone (39).

16. HF-Generator (10) nach einem der vorhergehenden Ansprüche, **dadurch gekennzeichnet, dass** der Ausgangsblock (11) über den zweiten Leistungskoppler (29) und den dritten Leistungskoppler (30) gegenüber dem Netzblock (14) gesichert ist.
